# EUROPÄISCHE PATENTSCHRIFT

(11) **EP 1 283 047 B1**
(45) Veröffentlichungstag und Bekanntmachung des Hinweises auf die Patenterteilung: **17.01.2007**
(21) Anmeldenummer: 00935752.6
(22) Anmeldetag: 29.02.2000
(51) Int. Cl.: A61K 35/16, A61K 38/02, A61P 15/10, A61P 25/16, A61P 25/18, A61P 25/28, A61P 27/16

(54) **METHODE ZUR HERSTELLUNG EINER BIOAKTIVEN SUBSTANZ AUS BLUTSERUM**
METHOD FOR PRODUCING A BIOACTIVE SUBSTANCE FROM BLOOD SERUM
PROCEDE DE PRODUCTION D'UNE SUBSTANCE BIOLOGIQUEMENT ACTIVE A PARTIR DU SERUM SANGUIN

(43) Veröffentlichungstag der Anmeldung: 12.02.2003
(73) Patentinhaber: OWEN Holding LTD, Douglas, Isle of Man (GB)
(72) Erfinder: OWEN Holding LTD, Douglas, Isle of Man (GB)
(74) Vertreter: Zwicker, Jörk
(86) Internationale Anmeldenummer: PCT/RU2000/000073
(87) Internationale Veröffentlichungsnummer: WO 2001/064228

(56) Entgegenhaltungen:
- EP-A- 0 542 303
- WO-A-92/06696
- DD-A- 228 738
- GB-A- 2 130 088
- RU-A- 97 119 627
- RU-C1- 2 097 072
- RU-C1- 2 104 044
- RU-C1- 2 136 292
- SU-A- 1 015 894
- US-A- 4 054 557

## Beschreibung

### Technisches Fachgebiet, zu dem die Erfindung gehört

Die präsentierte Erfindung gehört zur Medizin und kann zur Gewinnung aus dem Blutserum einer aktiven Substanz verwendet werden, die sich bei einigen Erkrankungen und Störungen des menschlichen und tierischen Organismus als nützlich erweist (Hörvermögen, Geschlechtsaktivität, räumliches Gedächtnis etc.).

### Stand der Technik

Weit bekannt sind Verfahren zur Gewinnung einer aktiven Substanz aus dem Blutserum, denen die Entnahme des Spenderblutes, die Inkubation sowie die Abscheidung nut anschließender Konservierung zugrunde liegen. Diese Verfahren setzen voraus die Gewinnung von Immunserum oder von Blutserum, das in Umgehung des Immunsystems wirkt, welches die Widerstandskraft des Organisamus gegen solche exogene und endogene Faktoren erhöht wie atmosphärischer Druck, Temperatur, Schwerkraft, Licht u. dgl. sowie gegen Hunger, Durst, Schlaf- und Sexbedürfnis u. dgl. (Japans Patent Nr.2123287, EP 0542303A2, Russlands Patent Nr.2096041, Russlands Patent Nr.2120301). Im zweiteren Falle erhält man das Serum vom Spender, der zuvor in einen bestimmten funktionalen Zustand gebracht wird; dabei werden, je nach der Einwirkungsart, Seren mit unterschiedlich gearteter biologischer Aktivität: myogen, somnogen, ophtalmugen, audioaktiv, thermoaktiv, diätaktiv, sexaktiv, antihypoxisch, Antialkohol- und Antinikotin-. Die bemerkenswerte Besonderheit der Seren, die in den Quellen (1) beschrieben sind, ist deren absolute Unbedenklichkeit (keine Toxizität); wie die Autoren feststellen, gibt es für diese Seren keine Gegenindikationen

Relativer Nachteil der genannten Seren besteht darin, dass der Anwender - darunter auch der behandelnde Arzt - in der Regel nicht weiß, mit welcher konkreter Substanz er zu tun hat, und ihm somit die Entscheidung für die eine oder andere Behandlungskur zur Zustandskorrektur des jeweiligen Patienten schwer fällt.

Das Anliegen der vorgestellten Erfindung war die Abscheidung einer neuen aktiven Substanz aus dem Blutserum, deren Identifizierung, d.h. Ermittlung von deren biologischer Aktivität, aber auch die Ermittlung der physikalisch-chemischen Eigenschaften, um dieses Produkt von anderen biologisch-aktiven Komponenten unterscheiden zu können, die aus dem Blutserum gewonnen wurden.

Die andere Aufgabe der Erfindung bestand darin, das Spektrum jener Quellen zu erweitern, aus denen sich die aktive Substanz gewinnen läßt, und somit die Möglichkeit zu finden, das Endprodukt zu verbilligen.

Eine weitere Aufgabe der Erfindung war die Entwicklung von praktisch anwendbaren Arzneimittelformen der aktiven Substanz.

Eine zusätzliche Aufgabe der Erfindung war die Ermittlung der Dosisintervallen der aktiven Substanz, die für die Zustandskorrektur des Patienten nützlich sind.

### Kurzbeschreibung des Wesens der Erfindung

Überraschend war, dass die Behandlung des Blutserums mit Gammastrahlen dazu führt, dass darin eine überaus aktive Substanz entsteht, die sich auf einige Funktionen des Organismus des Patienten positiv auswirken. Dies war um so mehr überraschend als bis dato laut der gängigen Meinung die ionisierende Strahlung von 25 kGy eine sterilisierende Dosis sei. Die genannte Dosis wird zur Sterilistion medizinischer Produkte in der Russischen Föderation empfohlen (Staatliche Pharmakopoe der UdSSR.// 1.Auflage. Moskau, Medizin-Verlag. - 1990 - Ausgabe 2. - S.19-24). Zugleich war bekannt (Martynow W.A. u.a. Einfluss der Strahlensterilisation auf die physikalisch-chemischen Eigenschaften der blutstillenden Gaze sowie auf die biochemische Aktivität der Proteinpräparate.//Radiazionnaya Medizina. Moskau, Verlag Atomisdat. 1972. Seiten 123-125; Gergely J., et. al. Studies of gamma-raz-irradiated human immunoglobulin G.//Radiosterilization of Medical Products,- 1967. - Vienna.- S. 115-124), dass das Blut und dessen Präparate sowie die Arzneimittel peptider Herkunft strahlenempfindlich und instabil sind und unter Einwirkung der ionisrenden Strahlen inaktiv werden. All das ließ nicht das Ergebnis erwarten, welches die Urheber der vorliegenden Erfindung bekommen haben.

Es stellte sich heraus, das wenn man venöses Blut der Tiere (insbesondere von Pferd und Mensch) oder arterielles Vogelblut im Laufe von etwa 24 Stunden unter niedrigen Temperaturen (ca. 4-8 ⁰C) inkubiert und anschließend nach der Blutgerinnselretraktion und -abscheidung ein Serum gewinnt, lyofilisiert und mit ca. 10 bis 40 kGy bestrahlt, so läßt sich eine aktive Substanz S (1, 2, 3) gewinnen, die einige Aktivitäten aufweist (darauf wird weiter unten eingegangen), die sich bei machen ungünstigen Zuständen des Patienten (darauf wird weiter unten eingegangen) als nützlich erweisen.

Laut Angaben der Massenspektrometrie entstehen in den Blutseren als Ergebnis der Behandlung Peptidenfraktionen, die zuvor im nativen Serum nicht nachzuweisen waren, wobei - je nach der Serumsart - die Eigenschaften der Peptide stark differieren.

Somit war das Ziel der vorliegenden Erfindung die Entwicklung eines neuen Verfahrens zur Gewinnung biologisch aktiver Substanz aus dem Blutserum.

Bei einem weiteren Ausführungsaspekt richtet sich die Erfindung auf die neue biologisch aktive Substanz, die aus dem Blutserum einiger Tiere und Vögel gewonnen wurde.

Im bevorzugten Durchführungsaspekt richtet sich die Erfindung auf die Substanz und deren Gewinnung aus dem Blutserum des Pferdes.

In einem weiteren Durchführungsaspekt richtet sich die Erfindung auf die Substanz und das Verfahren für deren Gewinnung aus dem Blutserum der Vögel.

In einem anderen Durchführungsaspekt richtet sich die Erfindung auf die Substanz und das Verfahren für deren Gewinnung aus dem Blutserum des Menschen.

Ein weiteres Ziel der vorliegenden Erfindung ist die Entwicklung einer Arzneimittelform für die neue biologisch aktive Substanz. Die Erfindung setzt diverse Arzneimittelformen voraus: darunter auch für perorale, parenterale, nasale und buccale Gabe, in Form von Suppositorien und für ähnliche Verabreichungsarten.

In der Erfindung ist die Verwendung von geeigneten und physiologisch vertretbaren Medien (wie z.B. distill. Wasser), Füllmitten (z.B. Kakaoöl) vorgesehen. Die biologisch aktive Substanz kann sowohl selbständig als auch in Verbindung mit anderen biologisch aktiven Mitteln verwendet werden.

Ein zusätzliches Ziel der Erfindung ist eine pharmazeutische Komposition, bei der die aktive Substanz gemäß der vorliegenden Erfindung als Agens auftritt. Dabei muß die pharmazeutische Komposition den aktiven Inhaltsstoff in einer Menge aufweisen, die ausreicht, um eine günstige Wirkung auf den Organismus des Patienten auszuüben, d.h. eine effektive Menge.

Ein weiteres Ziel der Erfindung ist die Ermittlung der effektiven Dosis der aktiven Substanz gemäß der vorliegenden Erfindung. Vermutlich liegt diese Dosis im Bereich von 0,15 bis 1000 mg/kg des Körpergewichtes des Patienten. Die konkrete Dosis soll offensichtlich der behandelnde Arzt ausgehend vom Zustand des Patienten, seinem Körpergewicht und der Behandlungskur festlegen.

**Die Erfindung wird weiter unten mit Abb. 1-3 erläutert,**
wo folgendes dargestellt ist:
Abb.1- das Spektrum des Blutserums der Vögel (S1) vor Bestrahlung (Kontrolle) und nach Bestrahlung (Versuch);
Abb.2 - - das Spektrum des Blutserums des Menschen (S3) vor Bestrahlung (Kontrolle) und nach Bestrahlung (Versuch);
Abb.3 - - das Spektrum des Blutserums des Menschen (S3) vor Bestrahlung (Kontrolle) und nach Bestrahlung (Versuch).

### Ausführliche Beschreibung der Erfindung

Nachfolgend wird die Erfindung in den bevorzugten Varianten der konkreten Ausführung eingehend geschildert. Dabei dürfen die angeführten Beispiele der aktiven Substanzen und Gewinnungsverfahren, der pharmazeutischen Kompositionen und Arzneimittelformen kein Grund zur Einschränkung der Ansprüche sein, sondern sind lediglich dazu gedacht, die Machbarkeit der Erfindung sowie die Umsetzung der genannten Zweckbestimmung(en) vorzuführen. Jeder Fachmann auf diesem Gebiet wird sich sicherlich davon überzeugen, dass für die hier angeführten Optionen der Erfindungsausführung zahlreiche Modifikationen vorgeschlagen werden können, die allesamt unter die Ansprüche fallen, die weiter unten in der Erfindungsformel geschildert sind.

### 1. Methodik zur Blutserum-Gewinnung von Vögeln (Substanz S-1), Pferden (Substanz S-2) und Mensch (Substanz S-3)

Zur Serumsgewinnung verwendete man das Blut der Vögel, der Pferde und des Menschen. Das Blut wurde aus punktierten Venen (Pferde, Mensch) und Arterien (Vögel) gesammelt. Das Blut wurde in Galsfläschchen gesammelt, die dann innerhalb von 24 Stunden unter einer Temperatur von 4-8 °C inkubiert wurde. Nach der Blutgerinnselretraktion wurden die Fläschchen im Laufe von 20-30 Minuten bei 3000 U/min zentrifugiert, das Serum wurde von Bluttgerinnseln getrennt und unter gewöhnlichen Verhältnissen lyofilisiert. Die Fläschchen mit dem lyofilisierten Serum wurden auf RZ-100M-Anlagen (Biotechnologija, Russland) in den Betriebsarten 19, 20, 30 oder 40 kGy unter Nutzung von Co⁶⁰ bestrahlt. Die auf diese Weise gewonnene Substanz S (1, 2, 3) wurde bei einer von 4-8 ⁰C gelagert.

### 2. Beweise für die stimulierende Wirkung der Gammastrahlung auf das Serum von Vögeln, Pferden und Mensch

### 2.1. VOGELSERUM (Substanz S-1)

Die Versuche wurden an Ratten der Linie WISTAR (Männchen mit Körpermasse von 220-240 g) durchgeführt.

Bei der ersten Versuchsreihe wurden die Tiere in 6 Gruppen je 20 Ratten eingeteilt:
1.Gruppe - den Ratten wurde eine physiologische Lösung verabreicht;
2.Gruppe - den Ratten wurde unbestrahltes Serum verabreicht;
3.Gruppe - den Ratten wurde mit 10 kGy bestrahltes Serum verabreicht;
4.Gruppe - den Ratten wurde mit 20 kGy bestrahltes Serum verabreicht;
5.Gruppe - den Ratten wurde mit 30 kGy bestrahltes Serum verabreicht;
6.Gruppe - den Ratten wurde mit 40 kGy bestrahltes Serum verabreicht;

Die physiologische Lösung, unbestrahltes Serum und die Substanz S-1 wurden intraperitoneal in einem Volumen von 1 ml verabreicht. Das Serum und die Substanz S-1 wurden in diesem Volumen jeweils in den Dosen von 15, 60, 100 und 120 mg/kg Körpermasse der Ratten gelöst. Sämtliche Stoffe wurden 30 Minuten vor dem Beginn der Versuchstests verabreicht. Die Vortests wurden 48 Stunden vor dem Beginn der Versuchstests durchgeführt.

Untersucht wurde die Einwirkung der Substanz S-1 auf die körperliche Ausdauer, ermittelt an der Schwimmzeit der Tiere unter Belastung (eine am Schwanz befestigte Last mit 30 g Masse). Dazu wurden die Ratten paarweise in ein mit Wasser gefülltes thermostatisches Gefäß ausgesetzt, das in zwei Teile getrennt war.

Die Wassertemperatur betrug +25 °C.

Die Lufttemperatur betrug +20 °C.

Ermittelt wurde die Schwimmzeit der Tiere bis zu den ersten Krampferscheinungen und bis zum endgültigen Schwimmverzicht und Abtauchen auf den Gefäßboden.

**TABELLE 1**

| **Einwirkung auf die körperliche Ausdauer der Strahlendosis der Substanz S-1 in einer Konzentration 100 mg/kg Körpermasse der Ratten** | | |
|---|---|---|
| Bestrahlungsverhältnisse | Vortest (ohne Substanz) | Versuchstest (nach Verabreichung der Substanz S-1) |
| Phys. Lösung (ohne Bestrahlung) | 4'50"± 30" | 4'59"± 18" |
| Serum ohne Bestrahlung | 4'33"± 19" | 4'40"± 29" |
| 10 kGy | 4'44"± 20" | 8'23"± 24" |
| 20 kGy | 4'44"± 20" | 8'51"±18"* |
| 30 kGy | 4'47"± 23" | 8'33"± 36"* |
| 40 kGy | 4'46"± 43" | 4'41"± 32"* |

| | | |
|---|---|---|
| Anmerkung: *- p <0,05 | | |

**TABELLE 2**

| **Einwirkung auf die körperliche Ausdauer der Substanz S-1 in einer Konzentration von 15, 60 und 120 mg/kg Körpermasse der Ratten** | | |
|---|---|---|
| **Bestrahlung bei 10 kGy** | | |
| Serumdosis | Vortest (ohne Substanz) | Versuchstest (nach Verabreichung der Substanz S-1) |
| Kontrolle | 4'45"± 27" | 4'54"± 22" |
| 15 mg/kg | 4'45"± 17" | 9'02"± 56"* |
| 60 mg/kg | 4'46"±31" | 7'16"± 53"* |
| 120 mg/kg | 4'52"±23" | 9,04"± 55"* |

| | | |
|---|---|---|
| Anmerkung: *-p<0,05 | | |

**TABELLE 3**

| **Einwirkung auf die körperliche Ausdauer der Substanz S-1 in einer Konzentration von 15, 60 und 120 mg/kg Körpermasse der Ratten** | | |
|---|---|---|
| **Bestrahlung bei 20 kGy** | | |
| Serumdosis | Vortest (ohne Substanz) | Versuchstest (nach Verabreichung der Substanz S-1) |
| Kontrolle | 4'45"± 27" | 4'54"± 22" |
| 15 mg/kg | 4'33'± 28" | 7'55"± 19"* |
| 60 mg/kg | 4'39"± 24" | 8'15"± 27"* |
| 120 mg/kg | 4'59"± 27" | 9,42"± 41"* |

| | | |
|---|---|---|
| Anmerkung: * - p <0,05 | | |

**TABELLE 4**

| **Einwirkung auf die körperliche Ausdauer der Substanz S-1 in einer Konzentration von 15, 60 und 120 mg/kg Körpermasse der Ratten** | | |
|---|---|---|
| **Bestrahlung bei 30 kGy** | | |
| Serumdosis | Vortest (ohne Substanz) | Versuchstest (nach Verabreichung der Substanz S-1) |
| Kontrolle | 4'45"± 27" | 4'54"± 24" |
| 15 mg/kg | 4'55'± 24" | 9'18"± 52"* |
| 60 mg/kg | 4'55"± 24" | 7'17"± 36"* |
| 120 mg/kg | 4'43"± 32" | 8,49"± 29"* |

| | | |
|---|---|---|
| Anmerkung: * - p <0,05 | | |

Aus der Tabelle 1 ist ersichtlich, dass die Verabreichung der aktiven Substany S-1 in einer Dosis von 100 mg/kg Körpermasse der Ratten bei einer Bestrahlung mit 10 kGy bereits statistisch aussagekräftige Erhöhung der körperlichen Ausdauer der Tiere mit sich bringt. Die Untersuchung der Dosis-Effekt-Abhängigkeit bei unterschiedlichen Bestrahlungsverhältnissen (Tabellen 2 und 3) erab, dass ein statistisch aussagekräftiger positiver Effekt ab einer Dosis unter 15 mg/kg nachweisbar ist.

### 2.2. PFERDESERUM (Substanz S-2)

Die Versuche wurden nach der Technologie gemäß Punkt 2.1. durchgeführt, ausgenommen die Verhältnisse der Gammbestrahlung. Die Strahlenbehandlung erfolgte bei 20 kGy. Unbestrahltes Serum und die Substanz S-2 wurden intraperitoneal in einer Dosis von 100 mg/kg Körpermasse der Ratten verabreicht.

**TABELLE 5**

| Bestrahlungsverhältnisse | Vortest (ohne Substanz) | Versuchstest (nach Verabreichung der Substanz S-2 |
|---|---|---|
| Serum ohne Bestrahlung | 4'0"± 23 | 5'19"± 35" |
| 20 kGy | 3'63"± 33" | 7'59"± 1'25"* |

| | | |
|---|---|---|
| Anmerkung: * - p <0,05 | | |

### 2.3. MENSCHLICHES SERUM (Substanz S-3)

Die Versuche wurden nach der Technologie gemäß Punkt 2.1. durchgeführt. Unbestrahltes Serum und die Substanz S-3 wurden in einer Dosis von 100 mg/kg Körpermasse der Ratten verabreicht.

**TABELLE 6**

| Bestrahlungsverhältnisse | Vortest (ohne Substanz) | Versuchstest (nach Verabreichung der Substanz S-1) |
|---|---|---|
| Serum ohne Bestrahlung | 4'49"±17" | 5'47"± 25" |
| 10 kGy | 4'43"± 16" | 7'08"± 12" |
| 20 kGy | 4'41"± 15" | 7'10"± 22"* |
| 30 kGy | 4'39"± 26" | 6'09"± 14"* |
| 40 kGy | 4'41"± 21" | 5'02"± 22"* |

| | | |
|---|---|---|
| Anmerkung: * - p <0,05. | | |

Die Daten der Tabellen 5 und 6 berechtigen zu einer definitiven Aussage über das Vorliegen eines positiven Effektes auf die körperliche Ausdauer der Ratten bei der Verabreichung der Substanzen S-2 und S-3 eben so wie im Falle der Substanz S-1.

### 2.4. STIMULIERUNG DER PPOLIFERATION EMBRYOBALER ZELLEN DES MENSCHLICHEN GEHIRNS DURCH DIE SUBSTANZ S-1

Mittel und Reagenzien. Wachstumsmedium: Nadel-Medium ("Igla"), modifiziert durch Dulbekko (DMEM) oder RPMI-1640 (Gribco, Grand Island), 10% des embryonalen Kuhserums (Sigma, USA). Die Stammlösung der Substanz S-1 20,0 mg/ml wird mittels Auflösen in DMEM zubereitet und in unterschiedlichen Konzentrationen in das Wachstums- oder Versuchsmedium mit Zellen auf eine 96-Grübchen-Kulturplanchette gebracht.

Versuchsmedium. Modifizierte Dulbekko-Nadel mit 0,58 g/l Glutamin, 50 mkg/l Gentamizin imd 0,2% Bullenserum-Albumin.

Hereingewinnung der Organe. Ein Embryo mit 10-12 Wochen Histazie, hereingewonnen mittels einer Kürette durch herkömmliche Abortmethode, wird in ein steriles Gefäß mit der Henks-Lösung gebracht, die 200 mkg/ml Gentamizin enthält. Das Gefäß wird abgeschlossen und maximal 24 Stunden bei einer T +4 °C in einem Kühlschrank gelagert. Das Embryo, das bei T +4 °C in einem Gefäß mit Flüssigkeit gelagert wurde, wird herausgenommen und auf eine PetriSchale im Laminarschrank gelegt, der vorher im Laufe von 60 Minuten mit UV-Strahlung behandelt wurde. Die dabei benutzten Instrumente sollen entweder durch Abkochen oder mittels 70% Äthanol sterilisiert sein. Das Gehirm wird in ein steriles Reagenzglas mit einer bis auf 4 °C abgekühlten Hibemationslösung (270 mM KH₂PO, 975 mM D-Sorbitol, 25 mM D-Glukose, 100 mM Natriumlaktat, 100 mkg/ml Gentamizin). Das Reagenzglas mit dem Organ wird bis zur nächsten Bearbeitungsphase maximal 8-9 in einem Kühlschrank bei einer T +4 °C gelagert.

Abscheidung der Gehirnzellen. Das Gehirn im Reagenzglas wird mittels Pipettierung resuspensiert, die grobe Suspension wird auf ein Nylonsieb mit 80-100 mkm-Poren ausgeschüttet und mit einem Pistill oder 10-ml-Spritzenplunger durchgerieben, wobei das Sieb ständig mit einer Hibernationslösung durchgespült wird. Die Suspension wird 5 min lang bei T +4 ⁰C und 1500 U/min zentrifugiert und in einer Hibemationslösung resuspensiert. Es wird die Zahl der lebendigen Zellen mittels Ausschließen von Trypanblau berechnet. Die Suspension wird erneut 5 min lang bei T +4 °C und 1500 U/min zentrifugiert und diesmal in einem Kultivierungsmedium (DMEM + 10% Embryoserum der Kühe + 10,0 mkg/ml Gentamizin) in einer Konzentration 3-10⁶ kl/ml resuspensiert. Die Suspension wird je 100 mkl in die Grübchen der 96-Grübchen-Kulturalplanchette, die vorher mit Kollagen bedeckt wurde. Die Kulturalplanchette wird für 24 Stunden in einen CO₂-Inkubator bei 37 °C, 5% CO₂ und 90% Feuchtigkeit getan. Alle 24 Stunden wereden in die Grübchen der 96-Grübchen-Kulturalplanchette jeweils 100 mkl Kultivierungsmedium hinzugefügt.

Messung der mitochondrialen Atmung nach der Mosman-Monks-Methode. In die 96-Grübchen-Kulturalplanchette werden die zu analysierenden Zellen im Wachstumsmedium gebracht, und 50 mkl 1mg/l MTT-Lösung in SFR bis zur Endkonzentration von 200 mkg/ml hinzugetan. Nach 2-4 Stunden, je nach der Aktivität der mitochondrialen Atmung der zu analysierenden Zellen, bilden sich am Boden der Grübchen violette im Wasser nicht lösbare Kristalle. Die 96-Grübchen-Kulturalplanchette mit den ausgefallenen Kristallen wird 10 min bei 1500 U/min zentrifugiert, die Flüssigkeit über dem Niederschlag wird vorsichtig abgesaugt, und danach 200 mkl DMSO zum Auflösen von Kristallen und zur Bildung einer gefärbten Lösung dazugegeben. Die optischen Dichtewerte werden bei einer Wellenlänge von 540 nm gemessen, was eine Kennzahl für die Aktivität der mitochondrialen Zellenatmung ist.

Versuchsbedingungen. Die Zellenkultur wird auf dem Wachstumsmedium bei 37 °C in einem CO₂-Inkubator in einer Atmosphäre mit 5% CO₂ und 95% Feuchtigkeit gezüchtet. Beim Versuch wird die Zellensuspension in Zetrifugen-Reagenzgläser ausgeschüttet, 5 min bei 1500 U/min in einem Bucket-Totor zentrifugiert, die Flüssigkeit über dem Niederschlag wird ausgegossen, der Zellenniederschlag mittels Pipettierung im Versuchsmedium resuspensiert. Danach wird die Zellensuspension in die Grübchen der 95-Grübchen-Kulturalplanchette hineingetan, und zuvor wird in diese Grübchen der zu analysierende Stoff in den nötigen Konzentration dazugetan. Die Kulturalplanchetten werden 24, 48, 72 Stunden oder länger in einem Volumen von 200 mkl/Grübchen inkubiert. Zu den Zellen wird eine MTT-Lösung (3-(4,5-Dimethylthiazol-2-el) -2,5-Diphenyltetra Zolium Bromid; Blau-Dimethyl) nach der Mosman-Methode hinzugefügt, um die mitochondriale Atmung zu messen. Bei der Direktzählung der Zellenanzahl mittels Färbung mit Trypanblau liegt der Rechenfehler +/-10-15%, während die Ermittlung der mitochondrialen Atmung eine Streubreite von +/-1,5-2,0% aufweist. Deswegen wird normalerweise als Zellenanzahl in der Probe die quantitative Schätzung des Niveaus der mitochondrialen Atmung genommen, weil dieser Wert mit höherer Genauigkeit gemssen werden kann. Um aussagekräftige Meßergebnisse zu bekommen, werden pro Meßpunkt jeweils 3-4 Grübchen gemessen.

### Untersuchungsergebnisse.

Bei sämtlichen getesteten Konzentrationen der biologisch aktiven Substanz S-1 (0,1-10,0 mg/ml) wurde die proliferative Aktivität des komplizierten Systems embryonaler Zellen des menschlichen Gehirns stimuliert (Tabelle 7). Der stimulierende Effekt von S-1 in einer Konzentration 0,2 mg/ml war vergleichbar mit dem stimulierenden Effekt von 0,1 ME/ml Insulin und bei einer Konzentration 1,0 mg/ml ähnlich der Wirkung von 1%-Albumin-Lösung.

**Tabelle 7**

| | Kontrolle | Substanz S-1 | | | | | 1% Alb | 0,1 ME/ml Ins | 1%Alb +0,1 ME/ml Ins | 1mg/ml S-1 +0,1 ME/ml Ins | S-1 1mg/ml + 0,5%Alb +0,1 ME/ml Ins |
|---|---|---|---|---|---|---|---|---|---|---|---|
| | | 10 mg/ml | 2 mg/ml | 1 mg/ml | 0,2 mg/ml | 0,1 mg/ml | | | | | |
| opt. Dichte | 0,054 | 0,359 | 0,298 | 0,22 | 0,161 | 0,123 | 0,269 | 0,163 | 0,45 | 0,33 | 0,239 |

| | | | | | | | | | | | |
|---|---|---|---|---|---|---|---|---|---|---|---|
| Anmerkung: S-1 - biologisch aktive Substanz Alb - Albumin Ins - Insulin opt. Dichte - optische Dichte | | | | | | | | | | | |

Eine derartige Reaktion der embryonalen Zellen des menschlichen Gehirms auf die Einwirkung der Substanz S-1 zeugt davon, dass in dieser Phase der Zytogenese von Gehirnzellen S-1 als trophischer Faktor füngiert. Dabei verweisen die zytomorphologischen Eigenschaften des untersuchten Gewebes auf die besondere Empfindlichkeit der astrozytarer Zellen gegenüber dieser Einwirkung.

Zugleich ergab die Analyse der kombinatorischen Wirkung von S-1 mit Albumin und Insulin, dass bei den genannten Substanzen konkurrente Wechselbeziehungen mit den Zellenrezeptoren bestehen, weil die gemeinsame Einwirkung aller drei Präparate in Konzentrationen, die bei separater Nutzung optimal waren, nicht den erwarteten Effekt zeigt.

Mehr noch: die gemeinsame Nutzung von 1,0 mg/ml S-1 und 0,1 ME/ml Insulin führt zu einer stärker ausgeprägten Proliferation als wenn man diesem Gemisch noch eine 0,5%-Albumin-Lösung beigibt.

Diese Daten sind ein Zeugnis dafür, dass S-1 einen wesentlichen Einfluss auf das Wachstum und die Lebenstätigkeit der Zellen ausübt, indem sie vermutlich auf die regulatorischen und trophischen Mechanismen einwirkt. Dabei können die Peptidenkomponenten der Substanz S-1 in ihrer Funktion als trophische Agenzien im Bestand der Aminsäuren (auch der Albumin-Amin säure) auftreten, während die regulatorischen Wirkungen auf der Ebene der Rezeptoren von äußeren Zellenmembranen und Mitochondrien zur Geltung kommen können, indem sie auf den Energieaustausch einwirken.

### PROTEIN-PEPTIDEN-ZUSAMMENSETYUNG DER SUBSTANZ S-1

Die Methodik der Mustervorbereitung für die Massenspektroskopie

Eine trockene Mustereinwaage wurde in 0,1%-Trifluoröthansäure (TFA) in einer Konzentration 10 mg/ml gelöst und 10 min lang bei 13 000 U/min zentrifugiert (20 cm Rotorradius).

Das Extrakt wurde über eine Umkehrphasensäule mit 1 ml LiChrosorbC18 (10 mkm) durchgelassen. Danach folgte die Stufenelution je 1 ml hintereinander: 10%, 30%, 80%, 100% Azetonitrilom (AN) mit 0,1% TFA-Gehalt. Die gewonnenen Fraktionen wurden einer massenspektrometrischen Analyse mittels MALDI TOF-Methode unterzogen.

### SUBSTANZ S-1

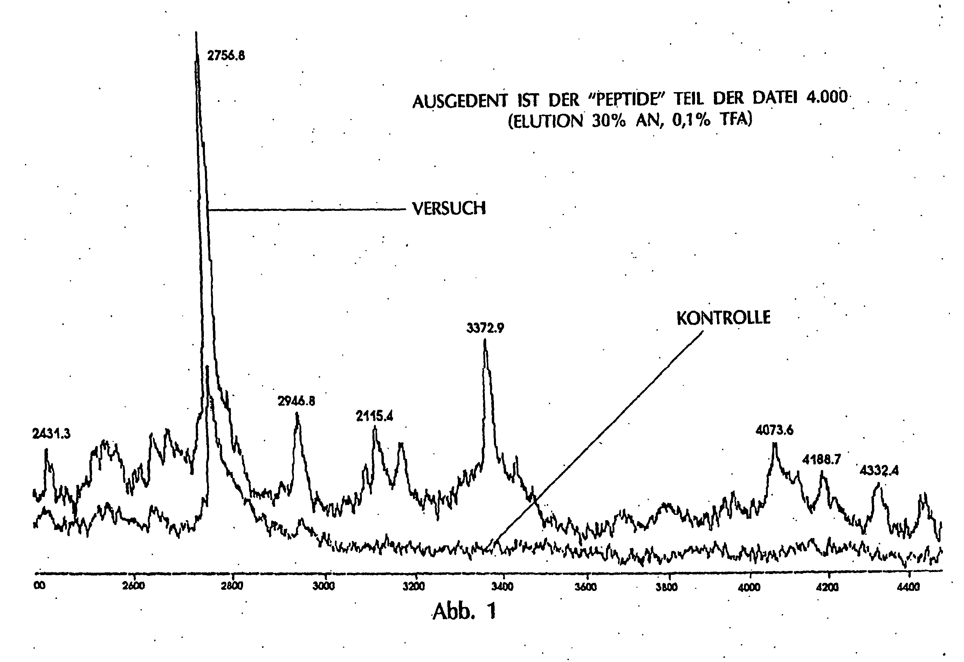

Nach der γ-Bestrahlung macht sich im Vogelserum ganz deutlich ein erhöhtes Niveau an niedrigmolekularen Peptiden bemerkbar (Abb. 1).

Die Differenzen der wichtigsten Spitzenwerte bei der Massenspektrometrie des Vogelserums vor und nach der γ-Bestrahlung sind in Tabelle 8 ausgewiesen.

**Tabelle 8**

| **MASSENSPEKTROMETRIE DER SUBSTANZ S-1** | |
|---|---|
| Vogelserum ohne γ-Bestrahlung (D) | Vogelserum nach der γ-Bestrahlung bei 20 kGy(D) |
| 66656,6-66808,8 | - |
| 43653,5-49599,3 | - |
| 14156,9-33708,5 | - |
| | 4073,8 |
| | 3115,4-3372,9 |
| | 2431,3-2946,8 |

### SUBSTANZ S-3

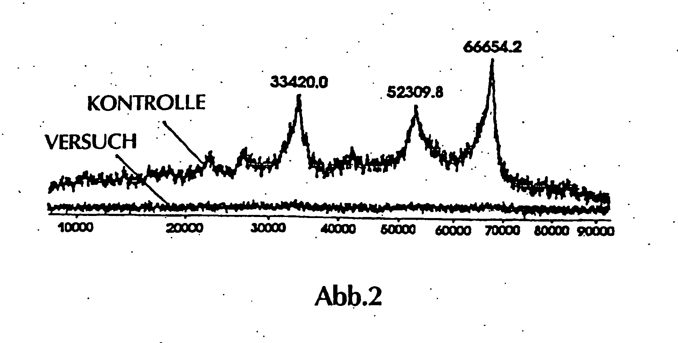

Beim menschlichen Serummuster lassen sich vor der γ-Bestrahlung in auffallender Menge Proteine nit Molekularmasse 33.4, 52.3, 66.6 kDa nachweisen: das erstere und das letztere netsprechen vermutlich dem Albumin, das in der Säule nicht sorbiert wurde. Nach der Bestrahlung verschwinden diese Spitzen (Abb.2).

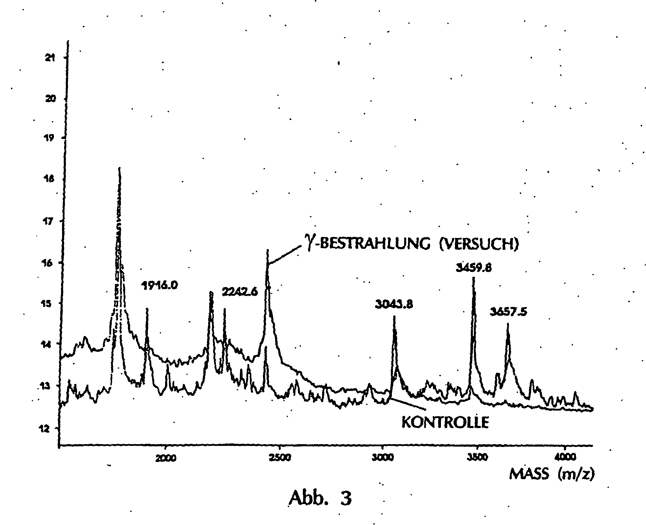

Starke Differenzen sind auch im Bereich der Molekularmassen von 2,0 bis 4,0 kDa bemerkbar, wobei nach der Bestrahlung niedrigmolekularen Peptide sich ganz deutlich nachweisen lassen (Abb.3).

Die Besonderheiten der Massenspektrometrie der Substanz S-3 sind in Tabelle 9 ausgewiesen.

**Tabelle 9**

| **MASSENSPEKTROMETRIE DER SUBSTANZ S-3** | |
|---|---|
| Menschliches Serum ohne γ-Bestrahlung (D) | Menschliches Serum nach der γ-Bestrahlung bei 20 kGy(D) |
| 66654,2 | - |
| 52309,8 | - |
| 22447,0-33420,0 | - |
| - | 7177,4-10571,3 |
| 1433,9-2434,2 | 2431,3-4332,4 |
| 823,1-1147,6 | 1208,8-2431,3 |
| | 895,0-1179,5 |

### THERAPEUTISCHE WIRKUNG DER SUBSTANZ S-1

### A. RÄUMLICHES GEDÄCHTNIS

Beim Versuch wurden Ratten der Linie WISTAR (Männchen mit Körpermasse von 220-240 g) genutzt.

Die Substanz S-1 wurde intraperitoneal in einer Dosis von 100 mg/kg der Körpermasse der Tiere 30 Minuten vor dem Testbeginn verabreicht.

Untersucht wurde die Einwirkung der Substanz S-1 auf das räumliche Gedächtnis in einem T-förmigen Labyrinth. 2 Tage vor den Tests wurden die Ratten an das Labyrinth gewöhnt und hatten die Möglichkeit, das Labyrinth im Laufe von 3 Minuten zu untersuchen. Keine der Ratten zeigte deutliche Bevorzugung für irgendwelche Seite. Die Ratten bekamen nichst zu essen während 36 Stunden vor den Tests, das Wasser war nach Bedarf da. Beim Versuch wurde ein einfaches Schema genutzt: das Futter befand sich immer rechts. Die Ratte wurde in die Startsektion ausgesetzt, und es wurden im Laufe 1 Minute deren Bewegungen über das Labyrinth registriert. Die Rückkehr in die Startsektion war möglich. Beim Versuch gab es 10 Anläufe. 2 Tage später wurden die Tests wiederholt.

Unter den 20 untersuchten Ratten (10 Kontrolltiere und 10 Versuchstiere) beobachtete man bei 10 Ratten den ersten Lauf nach links und bei 10 anderen nach rechts. Im folgenden verteilten sich die Laufrichtungen wie folgt (Tabelle 10).

**Tabelle 10**

| Gruppen | Linksläufe | | Rechtsläufe | | Kein Zugang |
|---|---|---|---|---|---|
| | Anzahl | Durchschn. Zeit | Anzahl | Durchschn. Zeit | |
| Kontrollgruppe | 29 | 14,1" | 55 | 22,7" | 16 |
| Versuchsgruppe | 22 | 21,7" | 68 | 24,0" | 10 |

**Tabelle 11**

| Gruppen | Linksläufe | | Rechtsläufe | | Kein Zugang |
|---|---|---|---|---|---|
| | Anzahl | Durchschn. Zeit | Anzahl | Durchschn. Zeit | |
| Kontrollgruppe | 21 | 24,9" | 55 | 23,2" | 24 |
| Versuchsgruppe | 18 | 20,4" | 75 | 17,7" | 77 |

Das Kriterium "3 richtige Zugänge nacheinander" erreichten während der wiederholten Tests 8 von 10 Kontrollratten und alle 10 Versuchsratten.

Somit kann man von der stimulierenden Wirkung der Substanz S-1 auf die Erlangung und Erhaltung der räumlichen fertigkeiten in einem T-förmigen Labyrinth, d.h. vom räumluichen Gedächtnis sprechen.

### 3. KORREKTUR DER PARKINSONISMUSERSCHEINUNGEN

Die Wirksamkeit der biologisch aktiven Substanz S-1 wurden an einem experimentellen Parkinson-Modell bewertet. Zur Simulation der Parkinsonschen Krankheit wurde Neurotoxin 6-Hydroxidophamin (6-GD), das die katecholaminergischen Terminals destrukturiert. 6-GD (6-Hidroxydophamin, 6-HODA) - 10 mkg in einem 10 mkl Volumen wurde steriotaxisch entlang den Koordinaten des Gehirnatlas der Fifkowa-Ratte Marschal im laufe von 10 Minuten in den kompakten Teil der schwarzen Substanz (SN) eingeführt.

6-GD wurde in der physiologischen Lösung gelöst, die 1% Askorbinsäure enthielt. Die eingeführte Lösung wurde mit Hilfe von Natriumbikarbonat-Lösung auf pH=7 gebracht. Die Applikation des Stoffes erfolgte unter Ärthernarkose zweimal (jeweils einmal binnen 24 Stunden), um stabile Verhaltensmuster zu bekommen.

Beim Versuch wurden Männchenratten der Linie Wistar mit der Körpermasse von 250-300 g genutzt.

Die Beobachtungen erfolgten im Laufe von 17 Tagen seit der ersten Injektion

Sämtliche Tiere wurden in 2 Gruppen je 20 Ratten eingeteilt. Den Tieren der ersten Gruppe wurden 1 Stunde nach der zweiten 6-GD-Applikation intraperitoneal die Subszanz S-1 verabreicht, die im distillierten Injektionswasser in einer Dosis 100 mg/kg gelöst war. Den Tieren der zweiten Gruppe wurde 1 ml distilliertes Wasser verabreicht, das 100 mg/kg gewöhnlich unbestrahltes Serum enthielt.

Die Symptome der Parkinsonschen Krankheit: Tremor, Pyloerektion, insilaterale zirkulatorische Drehbewegungen - wurden mit Expertenmethode bewertet. Diese Effekte lassen sich bei den Kontrolltieren im Laufe von 3 Tagen nach der letzten Applikation der 6-GD-Lösung nachweisen. Außerdem werden die beschriebenen Effekte im Laufe weiterer 12-14 Tage gewaltsam (taktile Einwirkung) bei den Tieren provoziert.

Die Verabreichung der Substanz hat jeweils die Erscheinungsformen der Parkinsonschen Krankheit wesentlich verändert. Die positive Einwirkung der Substanz S-1 auf die Parkinsonschen Merkmale sind in Tabelle 12 ausgewiesen.

**Tabelle 12**

| | | |
|---|---|---|
| Die typischen Merkmale der Parkinsonschen Krankheit | Kontrolle | Substanz S-1 |
| Tremor, Pyloerektion und zirkulatorische Drehbewegungen | 72 Stunden | 24 Stunden |
| Taktile Provokation der Merkmale der Parkinsonschen Krankheit | 12-14 Tage | bis 9 Tage |

### DIE THERAPEUTISCHE AKTIVITÄT DER SUBSTANZ S-2

Beim Versuch wurden Ratten der Linie WISTAR (geschlechtsreife Männchen und Weibchen mit Körpermasse von 220-240 g) genutzt.

### Intraperitoneale Verabreichung der Substanz S-2

Die Männchen wurden in 4 Gruppen je 10 Ratten eingeteilt. Aus der Verbindung dieser Gruppen wurden 4 Gruppen eingeteilt - KK (Kontrollmännchen und Kontrollweibchen), KO¹ (Kontrollmännchen und Versuchsweibchen), OK (Versuchsmännchen und Kontrollweibchen), und OO (Versuchsmännchen und Versuchsweibchen). Zuerst erfolgten Vortests. Danach wurde den Männchen eine 15 Tage lange Erholung gegönnt. Die Weibchen wurden jeweils nur einmal genutzt. Die Substanz S-2 wurde 30 Minuten vor dem Beginn der Versuchstests verabreicht. Den Kontrolltieren wurde 1 ml gewöhnliches Pferdeserum verabreicht.
¹O steht für das russische "Opyt" (Versuch)

Das Männchen wurde für 15 Minuten in einen getrennten Käfig ausgesetzt, danach wurde ein Weibchen im Estrus-Zustand (ermittelt an dem Zustand der äußeren Heschlechtsorgane) in denselben Käfig getan.

Während des Versuchs wurden folgende Kennziffern registriert:
- latente Periode der ersten Aussetzung (LP der Aussetzung);
- latente Periode der ersten Intromission (LPIM);
- die Zahl der Intromissionen (IM-Zahl);
- die Zahl der Aussetzungen ohne Intromissionen (Zahl "leerer Aussetzungen");
- latente Periode der Ejakulation (LP der Ejakulation);
- refraktäre Periode nach der Ejakulation (PERP);
- die Zahl der Ejakulationen im Laufe des Versuchs (Zahl der Ejakulationen).

**TABELLE 13**

| **Kennziffern der kopulativen Aktivität der Männchen aus der KK-Gruppe** | | | | | | | |
|---|---|---|---|---|---|---|---|
| | LP der Aussetzung | Zahl der "leeren Aussetzungen") | LP IM (sek) | IM-Zahl | LP der Ejakulation (sek) | PERP (sek) | Zahl der Ejakulationen |
| Kontrolle | 7,5±1,0 | 7,0±1,0 | 11,8±0,9 | 10,7±1,4 | 574±97 | 455±43 | 2-4 |
| Versuch | 7,6±1,1 | 7,1±0,8 | 11,9±0,9 | 10,7±0,9 | 560±90 | 453±44 | 2-4 |
| Differenz (%) | +1% | +1% | +1% | 0% | -2% | +0% | |

**TABELLE 14**

| **Kennziffern der kopulativen Aktivität der Männchen aus der KO-Gruppe** | | | | | | | |
|---|---|---|---|---|---|---|---|
| | LP der Aussetzung | Zahl der "leeren Aussetzungen") | LP IM (sek) | IM-Zahl | LP der Ejakulation (sek) | PERP (sek) | Zahl der Ejakulationen |
| Kontrolle | 7,5±1,0 | 6,3±0,6 | 11,9±0,9 | 10,9±0,7 | 593±92 | 444±46 | 2-5 |
| Versuch | 7,5±1,0 | 6,6±0,7 | 11,8±1,1 | 10,9±1,1 | 591±83 | 437±44 | 2-4 |
| Differenz (%) | +0% | +5% | -1% | 0% | 0% | -2% | |

**TABELLE 15**

| **Kennziffern der kopulativen Aktivität der Männchen aus der OK-Gruppe** | | | | | | | |
|---|---|---|---|---|---|---|---|
| | LP der Aussetzung | Zahl der "leeren Aussetzungen") | LP IM (sek) | IM-Zahl | LP der Ejakulation (sek) | PERP (sek) | Zahl der Ejakulationen |
| Kontrolle | 7,6±1,1 | 6,7±0,6 | 11,9±0,8 | 11,0±0,8 | 590±87 | 462±44 | 1-4 |
| Versuch | 7,6±1,1 | 4,3±1,0 | 9,1±0,7 | 15,0±0,8 | 591±76 | 370±39 | 4-6 |
| Differenz (%) | -29% | -36% | -24% | +36% | 0% | -20% | |

**TABELLE 16**

| **Kennziffern der kopulativen Aktivität der Männchen aus der 00-Gruppe** | | | | | | | |
|---|---|---|---|---|---|---|---|
| | LP der Aussetzung | Zahl der "leeren Aussetzungen") | LP IM (sek) | IM-Zahl | LP der Ejakulation (sek) | PERP (sek) | Zahl der Ejakulationen |
| Kontrolle | 7,3±0,9 | 6,6±0,8 | 11,8±0,9 | 10,9±1,3 | 585±85 | 459±39 | 1-4 |
| Versuch | 4,9±0,9 | 4,3±0,6 | 9,1±1,0 | 15,0±0,5 | 588±81 | 368±37 | 4-7 |
| Differenz (%) | -33% | -35% | -23% | +38% | 0% | -20% | |

Somit hat die intraperitoneale Verabreichung dem Weibchen der Substanz S-2 von Hengsten so gut wie keinen Einfluss auf die kopulative Aktivität des Männchens, während die Verabreichung der S-2 Substanz dem Männchen die Zahl der Intromissionen und Ejakulationen im Versuch erhöht sowie die refraktäre Periode nach der Ejakulation (PERP) verkürzt.

### Intragastricale Verabreichung der Substanz S-2

Die Männchen wurden in 4 Gruppen je 10 Ratten eingeteilt. Zuerst erfolgten Vortests. Danach wurde den Männchen eine 15 Tage lange Erholung gegönnt.

Die Weibchen wurden jeweils nur einmal genutzt. Die Substanz wurde 60 Minuten vor dem Beginn der Versuchstests verabreicht. Es wurden folgende Dosen verwendet: 300 mg/kg, 500 mg/kg und 1000 mg/kg. Den Kontrolltieren wurden 2 ml physiologische Lösung verabreicht.

Das Männchen wurde für 15 Minuten in einen getrennten Käfig ausgesetzt, danach wurde ein Weibchen im Estrus-Zustand (ermittelt an dem Zustand der äußeren Heschlechtsorgane) in denselben Käfig getan.

Während des Versuchs wurden folgende Kennziffern registriert:
- latente Periode der ersten Aussetzung (LP der Aussetzung);
- latente Periode der ersten Intromission (LPIM);
- die Zahl der Intromissionen (IM-Zahl);
- refraktäre Periode nach der Ejakulation (PERP);
- die Zahl der Ejakulationen im Laufe des Versuchs (Zahl der Ejakulationen).

**TABELLE 17**

| **Kennziffern der kopulativen Aktivität der Männchen aus der Kontrollgruppe** | | | | | |
|---|---|---|---|---|---|
| | LP der Aussetzung | LP IM (sek) | IM-Zahl | PERP (sek) | Zahl der Ejakulationen |
| Kontrolle | 9,6±0,9 | 16,3±1,0 | 7,6±1,7 | 558±46 | 2-5 |
| Versuch | 9,7±0,7 | 16,2±0,9 | 7,9±1,2 | 543±57 | 2-5 |
| Differenz (%) | +1% | -1% | +4% | -3% | |

**TABELLE 18**

| **Kennziffern der kopulativen Aktivität der Männchen aus der Gruppe mit der Dosis 300 mg/kg** | | | | | |
|---|---|---|---|---|---|
| | LP der Aussetzung | LP IM (sek) | IM-Zahl | PERP (sek) | Zahl der Ejakulationen |
| Kontrolle | 9,8±1,0 | 15,9±0,8 | 8,1±1,2 | 561±43 | 2-5 |
| Versuch | 8,1±0,8 | 13,0±1,0 | 11,7±1,6 | 422±51 | 3-6 |
| Differenz (%) | -17% | -18% | +44% | -25% | |

**TABELLE 19**

| **Kennziffern der kopulativen Aktivität der Männchen aus der Gruppe mit der Dosis 500 mg/kg** | | | | | |
|---|---|---|---|---|---|
| | LP der Aussetzung | LP IM (sek) | IM-Zahl | PERP (sek) | Zahl der Ejakulationen |
| Kontrolle | 9,9±1,0 | 16,1±0,9 | 7,7±1,3 | 523±61 | 2-4 |
| Versuch | 8,1±0,8 | 13,5±0,8 | 11,8±1,8 | 397±48 | 3-7 |
| Differenz (%) | -18% | -16% | +53% | -24% | |

**TABELLE 20**

| **Kennziffern der kopulativen Aktivität der Männchen aus der Gruppe mit der Dosis 1000 mg/kg** | | | | | |
|---|---|---|---|---|---|
| | LP der Aussetzung | LP IM (sek) | IM-Zahl | PERP (sek) | Zahl der Ejakulationen |
| Kontrolle | 9,7±0,9 | 16,1±1,0 | 7,7±1,6 | 541±39 | 2-5 |
| Versuch | 7,1±0,8 | 11,4±0,9 | 11,9±2,1 | 384±55 | 3-7 |
| Differenz (%) | -27% | -29% | +55% | -29% | |

Somit erhöht die intragastricale Verabreichung der Substanz S-2 die Zahl der Intromissionen und Ejakulationen im Versuch und außerdem verkürzt die latente Periode der Aussetzungen und Intremissionen sowie die PERP-Kennziffer.

Schlußfolgerung. Die Substanz S-2 stimuliert die geschlechtliche Aktivität der Versuchsratten (Männchen). Diese Substanz hat keinen Einfluss auf die geschlechtliche Aktivität der Weibchen.

### DIE THERAPEUTISCHE AKTIVITÄT DER SUBSTANZ S-3

Beim Versuch wurden Ratten der Linie WISTAR (Männchen mit Körpermasse von 240-260 g) genutzt.

Die Ratten wurden einzeln in Versuchskammern ("shuttle-boxes") hereingetan, die mit einer Trennwand in zwei Teile geteilt waren. Der vorhandene Schallerzeuger gestattete es, einen Schallerreger mit einer Frequenz von 7 kHz und Leistung von 1 bis 10 dB zu präsentieren. 84 Ratten befanden sich in den Kammern in 10 Versuchen - je 20 Präsentationen pro Versuch. Bei jeder Präsentation wurde jeweils ein Schallerreger mit 2 dB-Leistung und 5 sek Dauer eingeschaltet. Die Zeitdauer jeder einzelnen Präsentation betrug 30 sek, die Zeitdauer des Versuchs 10 Minuten.

Den Kontrolltieren (43 Ratten) wurde im Laufe von 5 Tagen je 1 ml gewöhnliches Spenderserum verabreichet, während man den Versuchstieren (42 Ratten) im Laufe von 5 Tagen täglich Streptomizin (Streptomizinsulfat aus der Produktion der Kiewer Fabrik für mediuinische Präparate) verabreicht wurde, und zwar gelöst im Injektionswasser in einer Dosis von 400 000 Einheiten j kg Körpergewicht des Tieres.

Nach den Tests wurde an die Versuchsgruppe der Ratten mit Hörstörungen intraperitoneal die Substanz S-3 (Bestrahlung bei 20 kGy) in einer Dosis von 10 mg/100 g Körpermasse der Tiere verabreicht. Die Reaktion auf den Schallerreger mit 2,0 dB-Leistung wurde 30 min nach der Verabreichung der Substanz S-3 eingeschätzt. Die Ergebnisse sind in Tabelle 21 ausgewiesen.

**Tabelle 21**

| **Der Einfluss der Substanz S-3 auf den Schallerreger der Tiere mit Hörstörung (in fiktiven Einheiten.)** | | |
|---|---|---|
| Kontrolle | Nach Verabreichung des Spenderserums bei einer Hörstörung | Nach Verabreichung der Substanz S-3 bei einer Hörstörung |
| 18,5±0,4 | 5,46±0,66 | 16,2±0,7 |

Somit hat die Substanz S-3 eine korrigierende Wirkung auf die Gehörverminderung, die beim Versuch durch die Streptomizingabe verursacht war.

## Patentansprüche

1. Verfahren zur Gewinnung biologisch aktiver Blutserum-Substanz, welches die Inkubation entnommenen Blutes, die Serumabscheidung, dessen Lyophilisation und anschließende Gammastrahlen-Behandlung bis zum Erscheinen von Peptiden der molekularen Masse von 2-4 kDa einschließt.

2. Verfahren gemäß Anspruch 1, wobei tierisches Blut verwendet wird.

3. Verfahren gemäß Anspruch 2, wobei Pferd oder Mensch als Tier fungiert.

4. Verfahren gemäß Anspruch 1, wobei Vogelblut verwendet wird.

5. Verfahren gemäß einem der vorstehenden Ansprüche, wobei die Blutinkubation bei einer Temperatur von ca- 4-8°C im Laufe von etwa 24 Stunden durchgeführt wird.

6. Verfahren gemäß einem der vorstehenden Ansprüche, wobei Co-60-Gammabestrahlung bei 10-30 kGy vorgenommen wird.

7. Blutserum-Substanz, die biologische Aktivität aufweist und Peptide mit molekularer Masse von 2-4 kDa enthält, herstellbar nach dem Verfahren gemäß Anspruch 1.

8. Blutserum-Substanz gemäß Anspruch 7, gewonnen aus dem venösen Blut eines Tieres.

9. Blutserum-Substanz gemäß Anspruch 8, wobei als Blut das Blut eines Pferdes genommen wird.

10. Blutserum-Substanz gemäß Anspruch 8, wobei als Blut das Blut eines Menschen genommen wird.

11. Blutserum-Substanz gemäß Anspruch 7, gewonnen aus Vogelblut.

12. Blutserum-Substanz gemäß einem der Ansprüche 7-11, die eine biologische Aktivität in einer Konzentration von 15 mg/kg bis 1 g/kg des Tiergewichtes (inkl. Mensch) aufweist.

13. Blutserum-Substanz gemäß einem der Ansprüche 7-11, die die körperliche Leistungskraft steigert, die Proliferation von embryonalen Gehirnzellen des Menschen stimuliert, die Parkinsonismuserscheinungen korrigiert, die Herausbildung und Aufrechterhaltung des räumlichen Gedächtnisses fördert, die geschlechtliche Aktivität der männlichen Individuen erhöht sowie eine korrigierende Wirkung auf das Hörvermögen bei dessen Nachlassen ausübt.

14. Pharmazeutische Zusammensetzung, die eine effektive Menge der Blutserum-Substanz gemäß Anspruch 7, herstellbar nach dem Verfahren gemäß Anspruch 1, sowie bei Bedarf zusätzlich auch ein pharmazeutisch vertretbares Medium, Füllungs- bzw. Lösungsmittel enthält.

15. Pharmazeutische Zusammensetzung gemäß Anspruch 14, die die körperliche Leistungskraft steigert, die Proliferation von embryonalen Gehirnzellen des Menschen stimuliert, die Parkinsonismuserscheinungen korrigiert, die Herausbildung und Aufrechterhaltung des räumlichen Gedächtnisses fördert, die geschlechtliche Aktivität der männlichen Individuen erhöht sowie eine korrigierende Wirkung auf das Hörvermögen bei dessen Nachlassen ausübt.

16. Pharmazeutische Zusammensetzung gemäß Anspruch 14 ausgeführt in Form einer Lösung, einer Tablette, eines Zäpfchens, eines Pulvers oder einer Kapsel.

17. Pharmazeutische Zusammensetzung gemäß Anspruch 14, wobei destilliertes Wasser als Lösungsmittel genutzt wird.

18. Pharmazeutische Zusammensetzung gemäß Anspruch 14, wobei Kakaoöl als Medium fungiert.

19. Verwendung der Substanz gemäß Anspruch 7 zur Erzeugung einer effektiven pharmazeutischen Zusammensetzung mit dem Ziel, die körperliche Leistungskraft des Patienten zu steigern.

20. Verwendung gemäß Anspruch 19, wobei die pharmazeutische Zusammensetzung in einer Dosis von 15 mg/kg bis 1 g/kg bezogen auf das Körpergewicht des Patienten wirksam ist.

21. Verwendung der Substanz gemäß Anspruch 7 zur Erzeugung einer effektiven pharmazeutischen Zusammensetzung mit dem Ziel, die Proliferation von embryonalen Gehirnzellen des Menschen zu stimulieren.

22. Verwendung gemäß Anspruch 21, wobei die genannte pharmazeutische Zusammensetzung in einer Dosis von 15 mg/kg bis 1 g/kg bezogen auf das Körpergewicht des Patienten wirksam ist.

23. Verwendung der Substanz gemäß Anspruch 7 zur Erzeugung einer effektiven pharmazeutischen Zusammensetzung mit dem Ziel, die Parkinsonismuserscheinungen zu korrigieren im Falle eines Patienten, der eine solche Korrektur braucht.

24. Verwendung gemäß Anspruch 23, wobei die genannte pharmazeutische Zusammensetzung in einer Dosis von 15 mg/kg bis 1 g/kg bezogen auf das Körpergewicht des Patienten wirksam ist.

25. Verwendung der Substanz gemäß Anspruch 7 zur Erzeugung einer effektiven pharmazeutischen Zusammensetzung mit dem Ziel, räumliches Gedächtnis bei einem Patienten herauszubilden, der das braucht.

26. Verwendung laut Anspruch 25, wobei die pharmazeutische Zusammensetzung in einer Dosis von 15 mg/kg bis 1 g/kg bezogen auf das Körpergewicht des Patienten wirksam ist.

27. Verwendung der Substanz gemäß Anspruch 7 zur Erzeugung einer effektiven pharmazeutischen Zusammensetzung mit dem Ziel, die geschlechtliche Aktivität eines männlichen Individuums zu erhöhen.

28. Verwendung gemäß Anspruch 27, wobei die pharmazeutische Zusammensetzung in einer Dosis von 15 mg/kg bis 1 g/kg bezogen auf das Körpergewicht des Patienten wirksam ist.

29. Verwendung der Substanz gemäß Anspruch 7 zur Erzeugung einer effektiven pharmazeutischen Zusammensetzung mit dem Ziel, das Hörvermögen bei dessen Nachlassen beim Patienten zu korrigieren.

30. Verwendung laut Anspruch 29, wobei die genannte pharmazeutische Zusammensetzung in einer Dosis von 15 mg/kg bis 1 g/kg bezogen auf das Körpergewicht des Patienten wirksam ist.

## Claims

1. A method for obtaining biologically active blood serum substance, which includes the incubation of blood taken, serum separation, the lyophilisation thereof and subsequent treatment with gamma-rays until the appearance of peptides of molecular weight of 2 - 4 kDa.

2. A method according to Claim 1, wherein animal blood is used.

3. A method according to Claim 2, wherein horse or human being acts as animal.

4. A method according to Claim 1, wherein bird blood is used.

5. A method according to one of the preceding claims, wherein blood incubation is carried out at a temperature of ca. 4-8°C in the course of approximately 24 hours.

6. A method according to one of the preceding claims, wherein Co 60-gamma radiation is performed at 10-30 kGy.

7. A blood serum substance which is biologically active, and which contains peptides with a molecular weight of 2-4 kDa, obtainable by the method of Claim 1.

8. A blood serum substance according to Claim 7, obtained from the venous blood of an animal.

9. A blood serum substance according to Claim 8, wherein the blood taken is the blood of a horse.

10. A blood serum substance according to Claim 8, wherein the blood taken is the blood of a human being.

11. A blood serum substance according to Claim 7, obtained from bird blood.

12. A blood serum substance according to one of Claims 7-11, which is biologically active in a concentration of 15 mg/kg up to 1 g/kg of the animal weight (including human beings).

13. A blood serum substance according to one of Claims 7-11, which increases the physical efficiency, which stimulates the proliferation of embryonic brain cells of the human being, which corrects symptoms of Parkinson's disease, which promotes development and maintenance of the spatial memory, which increases male sexual activity, and which exerts a correcting influence upon the hearing capacity if it is in decline.

14. A pharmaceutical composition which contains an effective quantity of the blood serum substance according to Claim 7, obtainable by the method of Claim 1, and which also, if required, contains additionally a pharmaceutically acceptable medium, filler or solvent.

15. A pharmaceutical composition according to Claim 14, which increases physical efficiency, which stimulates proliferation of embryonic brain cells of the human being, which corrects symptoms of Parkinson's disease, which promotes development and maintenance of the spatial memory, which increases male sexual activity, and which exerts a correcting effect upon the hearing capacity if it is in decline.

16. A pharmaceutical composition according to Claim 14, in the form of a solution, a tablet, a suppository, a powder or a capsule.

17. A pharmaceutical composition according to Claim 14, wherein distilled water is used as solvent.

18. A pharmaceutical composition according to Claim 14, wherein cocoa oil acts as medium.

19. Use of the substance according to Claim 7 for the production of an effective pharmaceutical composition with the objective of increasing the physical efficiency of the patient.

20. Use according to Claim 19, wherein the pharmaceutical composition is effective in a dose of 15 mg/kg to 1 g/kg in relation to the body weight of the patient.

21. Use of the substance according to Claim 7 for the purpose of producing an effective pharmaceutical composition with the objective of stimulating proliferation of embryonic brain cells in the human being.

22. Use according to Claim 21, wherein the afore-mentioned pharmaceutical composition is effective in a dose of 15 mg/kg to 1 g/kg, in relation to the body weight of the patient.

23. Use of the substance according to Claim 7 for the production of an effective pharmaceutical composition with the objective of correcting symptoms of Parkinson's disease in the case of a patient who needs such correction.

24. Use according to Claim 23, wherein the afore-mentioned pharmaceutical composition is effective in a dose of 15 mg/kg to 1 g/kg in relation to the body weight of the patient.

25. Use of the substance according to Claim 7 for the production of an effective pharmaceutical composition with the objective of developing spatial memory in a patient who needs it.

26. Use according to Claim 25, wherein the pharmaceutical composition is effective in a dose of 15 mg/kg to 1 g/kg in relation to the body weight of the patient.

27. Use of the substance according to Claim 7 for the production of an effective pharmaceutical composition with the objective of increasing male sexual activity.

28. Use according to Claim 27, wherein the pharmaceutical composition is effective in a dose of 15 mg/kg to 1 g/kg in relation to the body weight of the patient.

29. Use of the substance according to Claim 7 for the production of an effective pharmaceutical composition with the objective of correcting the hearing capacity if it is in decline in the patient.

30. Use according to Claim 29, wherein the afore-mentioned pharmaceutical composition is effective in a dose of 15 mg/kg to 1 g/kg in relation to the body weight of the patient.

## Revendications

1. Procédé de production d'une substance de sérum sanguin biologiquement active, comportant l'incubation de sang prélevé, la sécrétion de sérum, sa lyophilisation, et un traitement consécutif par des rayons gamma jusqu'à l'apparition de peptides d'une masse moléculaire de 2 à 4 kDa.

2. Procédé selon la revendication 1, où on utilise du sang animal.

3. Procédé selon la revendication 2, où le cheval ou l'homme est l'animal.

4. Procédé selon la revendication 1, où on utilise du sang d'oiseau.

5. Procédé selon une des revendications précédentes, où l'incubation de sang est effectuée pendant environ 24 heures à une température entre environ 4 à 8 °C.

6. Procédé selon une des revendications précédentes, où on effectue une irradiation aux rayons gamma Co-60 à 10 à 30 kGy.

7. Substance de sérum sanguin, présentant une activité biologique et comportant des peptides d'une masse moléculaire de 2 à 4 kDa, susceptible d'être obtenue par le procédé selon la revendication 1.

8. Substance de sérum sanguin selon la revendication 7, produite à partir du sang veineux d'un animal.

9. Substance de sérum sanguin selon la revendication 8, où on utilise comme sang le sang d'un cheval.

10. Substance de sérum sanguin selon la revendication 8, où on utilise comme sang le sang d'un homme.

11. Substance de sérum sanguin selon la revendication 7, produite à partir du sang d'oiseau.

12. Substance de sérum sanguin selon les revendications 7 à 11, présentant une activité biologique d'une concentration allant de 15 mg / kg jusqu'à 1 g / kg du poids de l'animal (l'homme inclus).

13. Substance de sérum sanguin selon les revendications 7 à 11, qui augmente la puissance corporelle, stimule la prolifération des cellules cérébrales embryonnaires de l'homme, corrige les phénomènes de parkinsonisme, favorise la formation et le maintien de la mémoire spatiale, augmente l'activité sexuelle des individus masculins et exerce un effet correctif sur la capacité auditive lorsqu'elle diminue.

14. Composition pharmaceutique, comportant une quantité effective de la Substance de sérum sanguin selon la revendication 7, susceptible d'être obtenue par le procédé selon la revendication 1 , ainsi en cas de besoin en plus également un médium, une matière de remplissage respectivement un solvant justifiable du point de vue pharmaceutique.

15. Composition pharmaceutique selon la revendication 14, qui augmente la puissance corporelle, stimule la prolifération des cellules cérébrales embryonnaires de l'homme, corrige les phénomènes de parkinsonisme, favorise la formation et le maintien de la mémoire spatiale, augmente l'activité sexuelle des individus masculins et exerce un effet correctif sur la capacité auditive lorsqu'elle diminue.

16. Composition pharmaceutique selon la revendication 14, réalisée sous la forme d'une solution, une tablette, un suppositoire, une poudre ou une capsule.

17. Composition pharmaceutique selon la revendication 14, où l'on utilise de l'eau distillée comme solvant.

18. Composition pharmaceutique selon la revendication 14, où de l'huile de cacao est le médium.

19. Utilisation de la substance selon la revendication 7 pour produire une composition pharmaceutique effective ayant pour but d'augmenter la puissance corporelle d'un patient.

20. Utilisation selon la revendication 19, où la composition est efficace pour une dose entre 15 mg / kg et 1 g / kg par rapport au poids corporel du patient.

21. Utilisation de la substance selon la revendication 7 pour produire une composition pharmaceutique effective ayant pour but de stimuler la prolifération des cellules cérébrales embryonnaires de l'homme.

22. Utilisation selon la revendication 21, où la composition est efficace pour une dose entre 15 mg / kg et 1 g / kg par rapport au poids corporel du patient.

23. Utilisation de la substance selon la revendication 7 pour produire une composition pharmaceutique effective ayant pour but de corriger les phénomènes de parkinsonisme dans le cas d'un patient qui a besoin d'une telle correction.

24. Utilisation selon la revendication 23, où la composition est efficace pour une dose entre 15 mg / kg et 1 g / kg par rapport au poids corporel du patient.

25. Utilisation de la substance selon la revendication 7 pour produire une composition pharmaceutique effective ayant pour but de former de la mémoire spatiale chez un patient qui en a besoin.

26. Utilisation selon la revendication 25, où la composition est efficace pour une dose entre 15 mg / kg et 1 g / kg par rapport au poids corporel du patient.

27. Utilisation de la substance selon la revendication 7 pour produire une composition pharmaceutique effective ayant pour but d'augmenter l'activité sexuelle chez un individu masculin.

28. Utilisation selon la revendication 27, où la composition est efficace pour une dose entre 15 mg / kg et 1 g / kg par rapport au poids corporel du patient.

29. Utilisation de la substance selon la revendication 7 pour produire une composition pharmaceutique effective ayant pour but de corriger la capacité auditive en cas de diminution chez le patient.

30. Utilisation selon la revendication 29, où la composition est efficace pour une dose entre 15 mg / kg et 1 g / kg par rapport au poids corporel du patient.
